# EUROPEAN PATENT APPLICATION

(11) **EP 3 998 085 A1**
(43) Date of publication of application: **18.05.2022**
(21) Application number: 21775920.8
(22) Date of filing: 23.03.2021
(51) Int. Cl.: A61K 47/69, A61K 47/60, A61K 47/61, A61K 31/519, A61K 31/41, A61P 35/00

(54) **PEGYLATED HEPARIN NANO-MICELLE LOADED WITH CARBOXYLIC ACID ANTI-TUMOR DRUG AND PREPARATION METHOD THEREFOR**

(30) Priority: 25.03.2020 CN 202010219320
(71) Applicant: Kindos Pharmaceuticals Co., Ltd., Chengdu, Sichuan 611731 (CN); Nanjing King-Friend Biochemical Pharmaceutical Co., Ltd., Nanjing, Jiangsu 210061 (CN)
(72) Inventor: LI, Fangnian, Chengdu, Sichuan 611731 (CN); TIAN, Shane Xinxin, Chengdu, Sichuan 611731 (CN)
(74) Representative: Kolster Oy Ab
(86) International application number: PCT/CN2021/082358
(87) International publication number: WO 2021/190495

(57) **Abstract**

The present invention discloses a PEGylated heparin nanomicelle loaded with a carboxylic acid anti-tumor drug. A drug loading system is a conjugate formed by loading the carboxylic acid anti-tumor drug onto a PEGylated heparin molecule. A natural polysaccharide heparin that is biodegradable, good in compatibility and high in availability is used as a drug carrier. By means of combining PEG modification and the carboxylic acid anti-tumor drug, nanoparticles show a remarkably enhanced anti-tumor therapeutic index and biological safety during in vivo treatment when compared with free drugs.

## Description

### FIELD OF THE PRESENT DISCLOSURE

The present invention relates to the technical field of medicine, in particular to a PEGylated heparin nanomicelle loaded with a carboxylic acid anti-tumor drug and a preparation method thereof.

### BACKGROUND OF THE PRESENT DISCLOSURE

At present, with the rapid development of anti-tumor drug carriers, various new drug delivery systems are constantly emerging, and nano-drug delivery systems based on macromolecular biomaterials emerge at the right moment. A multifunctional drug delivery system developed by combining macromolecular biomaterials with micro-molecules drugs through physical embedding or chemical bonding. Utilizing an EPR effect of macromolecular carrier materials on solid tumors, macromolecular carrier materials can selectively enrich drugs at tumor sites to realize passive targeting. The controllable drug release of tumor micro-ambient intelligence response can be realized through specific physiological characteristics of the tumor sites compared with normal tissues, such as the pH value, the GSH level or the concentration of specific enzymes, so as to further improve the therapeutic effect of chemotherapy drugs and reduce toxicity and side effects. In addition, it is demonstrated that a nano-drug delivery system can effectively improve the delivery of chemotherapy drugs into cells by antagonizing or counteracting the active pumping-out of drugs by tumor cells, thereby reducing the drug resistance of tumor cells and improving the treatment. The emergence of these new drug delivery systems is expected to realize development and application of new anti-tumor drug formulations.

In recent years, macromolecular drug delivery systems such as dendrimers, polymers or polymer micelles, liposomes and so on are widely studied. Although a lot of researches on nano-drug carriers have been reported, there is still a problem of poor biocompatibility of carriers. Some carriers will be cleared by a reticuloendothelial system (RES) after entering the body, while others can't pass through intracellular barriers such as cell membranes and nuclear membranes, so that they can hardly act on the target sites. Therefore, developing drug carriers with target recognition to transport drugs to target tumor cells and tumor tissues to specifically kill cancer cells, is the primary objective in research of nano-preparations.

### SUMMARY OF THE PRESENT DISCLOSURE

Based on existing problems in the study of nano-preparations, the present invention provides a PEGylated heparin nanomicelle loaded with a carboxylic acid anti-tumor drug. A natural polysaccharide heparin that is biodegradable, and has good compatibility and high availability, is used as a drug carrier. By means of combining PEG modification and the carboxylic acid anti-tumor drug, nanoparticles show a remarkably enhanced anti-tumor therapeutic index and biological safety during in vivo treatment when compared with free drugs.

In order to achieve the purpose of the present invention, the technical solution adopted by the present invention is as follows:

A PEGylated heparin nanomicelle loaded with a carboxylic acid anti-tumor drug, wherein a drug loading system is a conjugate formed by loading the carboxylic acid anti-tumor drug onto a PEGylated heparin molecule; and a specific structure is as follows:

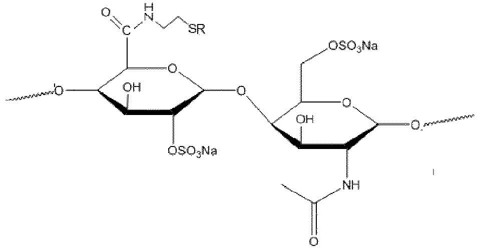

Where: R is a PEG group and a D group;

The PEG group is: 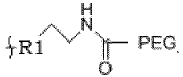 , and this group is an acyl group connected with a hydroxyl group at the end of polyethylene glycol via an ester bond,

Where: R1 is

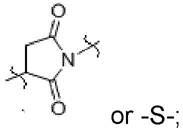

The structure of the D group is: 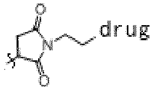 , and this group is connected with a carboxyl group in a drug molecule via an ester bond.

The heparin nano-drug loading system of the present invention forms the nanomicelle and loads the drug in the micelle, so that metabolic kinetics of the drug can be changed, effect kinetics of the drug can be improved, a usage amount of the drug can be reduced, and compliance of a patient can be improved. Targeting a target site accurately not only can increase a therapeutic effect, but also can reduce unnecessary side effects. The carrier heparin is of an endogenous structure, with a large safe dose; through combination of chemical bonds with the drug molecule, separation of the drug and ligands before use is avoided; and meanwhile, after reaching the target site, the drug molecule is specifically hydrolyzed to produce an effect, which improves targeting performance and safety.

Polyethylene glycol (PEG) can enhance water solubility of materials and stability of plasma protein, reduce immunogenicity at the same time. PEG is used to modify the heparin nano-carrier, so as to reduce nonspecific cell phagocytosis of the nano-carrier by a mononuclear phagocyte system (MPS), and meanwhile, a circulating half-life of nano-particles can be adjusted.

The carrier is of a water-soluble heparin structure, which is converted to a water-oil amphiphilic structure after the PEG is introduced; then a flexible ethyl sulfhydryl chain is introduced as a linkage, so that it not only reduces steric hindrance of a heparin sugar ring to subsequent conjugated compounds, but also is advantageous for adjusting a distribution state of the drug molecule in the micelle; and with a sulfhydryl as a binding site, types of the drug molecule to be conjugated can be increased.

The PEG of the present invention is mPEG2000(MeO-PEG₂₀₀₀-OH).

According to the present invention, the D group is connected with an aliphatic carboxyl group in the drug molecule via ester bonds, with the ease for synthesis.

The present invention further provides a preparation method of a PEGylated heparin nanomicelle loaded with a carboxylic acid anti-tumor drug, which comprises the following synthetic scheme:
(1) preparing a PEG derivative; 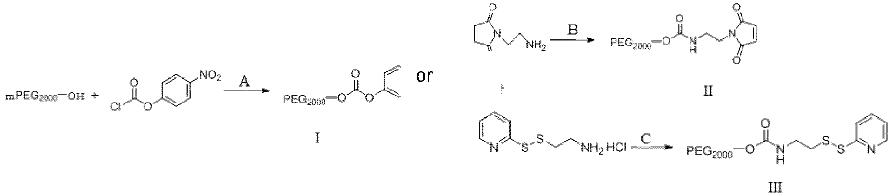
(2) preparing an intermediate A; 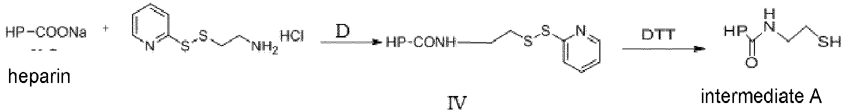
(3) reacting a carboxylic acid drug with Maleic-NH₂ to obtain a derivative of the carboxylic acid drug;
(4) reacting the intermediate A with the prepared derivative of the carboxylic acid drug to obtain a heparin loaded with the carboxylic acid drug;
(5) reacting the heparin loaded with the carboxylic acid drug with the PEG derivative to obtain a PEGylated heparin nanomicelle loaded with the drug.

In each of the reactions A, B and C of step (1), a catalyst DIEA is added. HCI generated in the reaction is neutralized, so as to facilitate the reaction with the provision of an alkaline environment.

In the reaction D, enoxaparin sodium is dissolved in a MeS buffer solution, and activated with addition of DMTMM; then S-(2-aminoethylthio)-2-thiopyridine that is dissolved in the MeS buffer solution is added dropwise to the system for reaction to obtain the intermediate A.

Preferably, a preparation method of the MeS buffer solution comprises the following steps: weighing quinoline-8-sulfonic acid and dissolving the quinoline-8-sulfonic acid in purified water, adding a sodium hydroxide solution dropwise to adjust the pH to 5.5, and making a metered volume to obtain the MeS buffer solution.

In the reaction of step (3), HOTu (O-[(ethoxycarbonyl)cyanomethyl-amine]-N,N,N',N'-tetramethylthiourea hexafluorophosphate) and DIEA are added to participate in the reaction. HOTu, as a condensing agent, is used to catalyze a condensation reaction of carboxyl and amine (hydroxyl) to form amide (ester).

In the reaction of step (4), the intermediate A reacts with the derivative of the carboxylic acid drug, and triethylamine as a catalyst is added. The sulfhydryl group is weakly acidic, and the ionization degree of the sulfhydryl group and the activity of nucleophilic addition reaction increase with addition of triethylamine.

The drug loading capacity of the nanomicelle of the present invention is preferably 4 wt%-15 wt%.

The present invention is advantageous in that:
1. The nano-drug loading system of the present invention is of a heparin structure, and heparin is of an endogenous polysaccharide structure of natural organisms, which can be injected per se for medicinal use, thereby avoiding metabolic toxicity of synthetic/semi-synthetic materials; and PEG modification of the carrier can reduce the rigidity of the carrier, improve the amphiphilic performance and micellization ability of the carrier, and address the problem of poor biocompatibility of existing macromolecular drug loading systems.
2. The drug loading system uses flexible aliphatic chains to connect the drug molecule, which is more conducive to self-assembly of the system into the appropriate micelle in water, so that the drug molecule can be stably wrapped inside the micelle, which can prevent degradation due to external factors or unnecessary metabolic inactivation, and address the problem of poor stability of the existing macromolecular drug loading systems; and using carbonate bonds to connect the carrier and the drug molecule can avoid separation of the drug molecule and the carrier under normal production/storage conditions, and after entering the body, the drug can be released at a designated site by catalysis of hydrolase, so as to realize targeting administration.
3. PEG is a polymer compound, and terminal OH activity thereof is not high, which is not conducive to subsequent reactions. According to the present invention, high-activity acyl chloride is used to react in a synthesis reaction, so that the intermediate I can be obtained in a high yield. P-nitrophenol is a good leaving group, which can be convenient for nucleophilic substitution in the subsequent reactions to connect target molecules.
4. Because the carboxyl group on the sugar ring of the heparin is influenced by steric hindrance of the sugar ring, the synthesis activity is low, which affects the yield. In the present invention, the flexible sulfhydryl aliphatic chain is introduced, which not only increases the reaction activity, but also helps to increase types of combination with drugs. The obtained intermediate A has 2-mercaptopyridine, which is an excellent leaving group, and will be replaced by nucleophilic substitution in case of more affinitive aliphatic sulfhydryl groups, which is convenient for obtaining of the PEGylated heparin through replacement of 2-mercaptopyridine in the PEG derivative reaction.
5. Because of the large steric hindrance and rigidity of the drug molecule, the drug molecule cannot be directly connected to the heparin main chain. In the present invention, the flexible aliphatic chain of maleimide is introduced as transition, and an α, β-conjugated unsaturated structure of maleimide can carry out an addition reaction with -SH of the heparin efficiently to connect two molecular fragments.

### BRIEF DESCRIPTION OF THE DRAWINGS

Fig. 1 shows morphological analysis of the appearance of a nano-drug loading system sample observed by a field emission transmission electron microscope (TEM), wherein a(1) is PEG-Py-HP-pemetrexed, and a(2) is PEG-Mal-HP-pemetrexed; b(1) is PEG-Py-HP-bendamustine, and b(2) is PEG-Mal-HP-bendamustine.
Fig. 2 is a diagram showing in vitro hemolysis experimental results of a nano-drug loading system of the present invention, wherein a(1) is PEG-Py-HP-pemetrexed and a(2) is PEG-Mal-HP-pemetrexed; and b(1) is PEG-Py-HP-bendamustine, and b(2) is PEG-Mal-HP-bendamustine.
Fig. 3 is a column chart showing in vivo experimental results of breast cancer cells under a nano-drug loading system of pemetrexed and bendamustine.
Fig. 4 is a column chart showing in vivo experimental results of non-small-cell lung cancer cells under a nano-drug loading system of pemetrexed and bendamustine.

### DESCRIPTION OF THE EMBODIMENTS

In order to explain the target technical solution of the present invention more clearly in detail, the present invention will be further described by related embodiments below. The following embodiments only specifically illustrate implementation methods of the present invention, and are not intended for limiting the scope of the present invention.

### Embodiment 1

Pemetrexed disodium is loaded on a PEGylated heparin molecules, with the following structure:

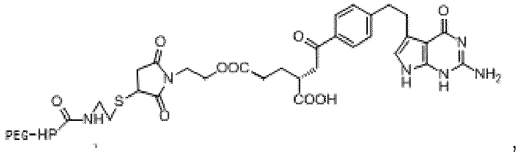

Where:

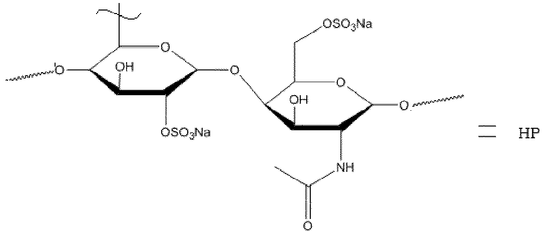

PEG-HP represents a PEGylated heparin polymer, with the structure as follows: 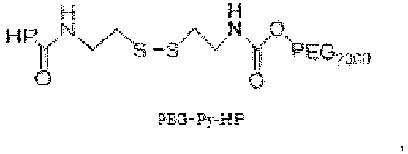 or

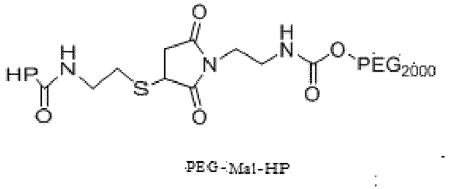

Where: the acyl group is connected with the hydroxyl group at the end of PEG via an ester bond.

### Embodiment 2

Bendamustine is loaded on a PEGylated heparin molecule, with the following structure:

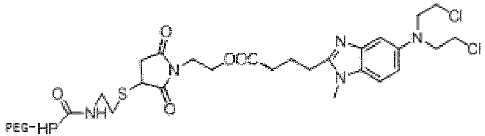

PEG-HP stands for a PEGylated heparin polymer with the structure as follows: 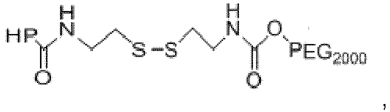 or

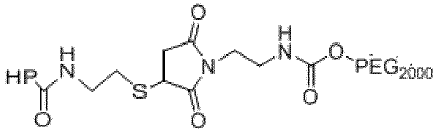

Where: the acyl group is connected with the hydroxyl group at the end of PEG via an ester bond.

### Embodiment 3

Raltitrexed is loaded on a PEGylated heparin molecule, with the following structure:

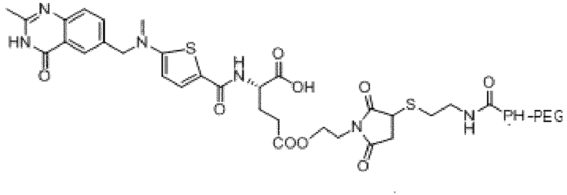

PEG-HP represents a PEGylated heparin polymer with the structure as follows: 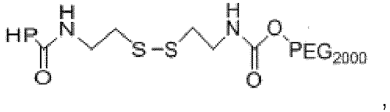 or

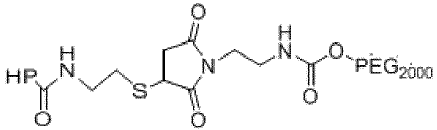

Where: the acyl group is connected with the hydroxyl group at the end of PEG via an ester bond.

### Embodiment 4

Methotrexate dihydrate is loaded on a PEGylated heparin molecule, with the following structure:

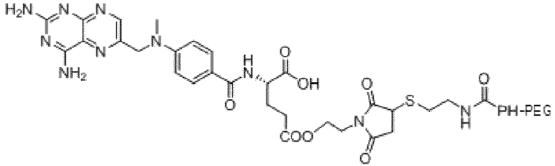

PEG-HP represents a PEGylated heparin polymer with the structure as follows: 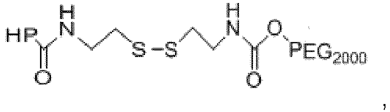 or

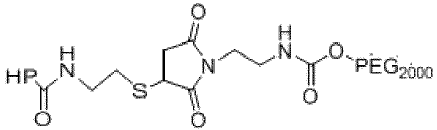

Where: the acyl group is connected with the hydroxyl group at the end of PEG via an ester bond.

### Embodiment 5

A preparation method of a PEGylated heparin nanomicelle loaded with a carboxylic acid anti-tumor drug comprises the following synthetic scheme:
(1) a PEG derivative is prepared; 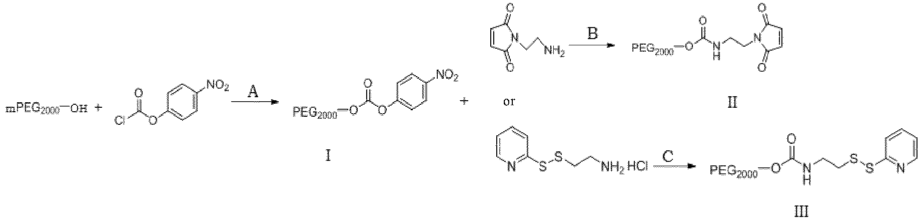
(2) an intermediate A is prepared; 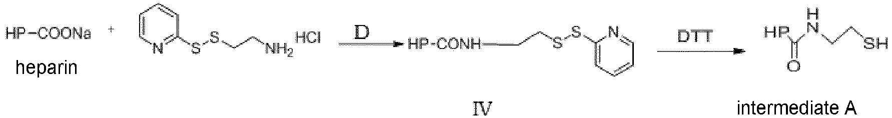 Acyl groups of some units in the heparin polymer react with 4-(4,6-Dimethoxy-1,3,5-triazin-2-yl)-4-methyl morpholinium chloride (DMTMM) to obtain a polymer IV, which is further desulfurized to obtain the intermediate A. The specific structural formula of intermediate A is as follows: 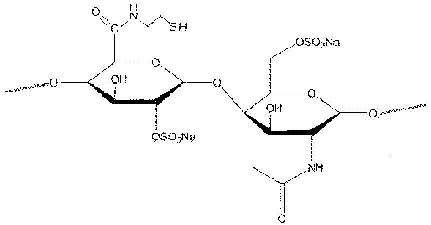
(3) a carboxylic acid drug reacts with Maleic-NH₂ to obtain a derivative of the carboxylic acid drug;
(4) the intermediate A reacts with the resultant derivative of the carboxylic acid drug to obtain a heparin loaded with the carboxylic acid drug;
(5) the heparin loaded with the carboxylic acid drug reacts with the PEG derivative, and -SH in the polymer unit of the intermediate A, which is not replaced by the drug molecule, reacts with the PEG derivative to obtain a PEGylated heparin nanomicelle loaded with the drug.

### Embodiment 6

The embodiment is based on Embodiment 3:
In the reactions A, B and C of step (1), a catalyst DIEA is added. HCI generated in the reaction is neutralized, and the reaction is facilitated by provision of an alkaline environment.
In the reaction D, enoxaparin sodium is dissolved in A quinoline-8-sulfonic acid (MeS) buffer solution, and activated with addition of 4-(4,6-Dimethoxy-1,3,5-triazin-2-yl)-4-methyl morpholinium chloride (DMTMM); in addition, then S-(2-aminoethylthio)-2-thiopyridine (Py-SS-NH2·HCl) that is dissolved in the MeS buffer solution is added dropwise to the system for reaction to obtain the intermediate A.
In the reaction of step (4), the intermediate A reacts with the derivative of the carboxylic acid drug, and triethylamine as a catalyst is added. The sulfhydryl group is weakly acidic, and the ionization degree of the sulfhydryl group and the activity of nucleophilic addition reaction increase by addition of triethylamine.

### Embodiment 7

Based on Embodiment 3, this embodiment is carried out as follows.

In the reactions A, B and C of step (1), a catalyst DIEA is added. HCI generated in the reaction is neutralized, and the reaction is facilitated by provision of an alkaline environment.

In the reaction D, enoxaparin sodium is dissolved in a quinoline-8-sulfonic acid (MeS) buffer solution, and activated with addition of 4-(4,6-Dimethoxy-1,3,5-triazin-2-yl)-4-methyl morpholinium chloride (DMTMM); in addition, S-(2-aminoethylthio)-2-thiopyridine (Py-SS-NH2·HCl) that is dissolved in the MeS buffer solution is added to the system dropwise for reaction to obtain the intermediate A.

A preparation method of the MeS buffer solution comprises the following steps: quinoline-8-sulfonic acid is weighed and dissolved in purified water, a sodium hydroxide solution is added dropwise to adjust the pH to 5.5, and a metered volume is made to obtain the MeS buffer solution.

In the reaction of step (3), HOTu and DIEA are added to participate in the reaction.

In the reaction of step (4), the intermediate A reacts with the derivative of the carboxylic acid drug, and triethylamine as a catalyst is added. The sulfhydryl group is weakly acidic, and the ionization degree of the sulfhydryl group and the activity of nucleophilic addition reaction are increased by addition of triethylamine.

### Embodiment 8

### Synthesis of a PEG derivative

Reaction A:

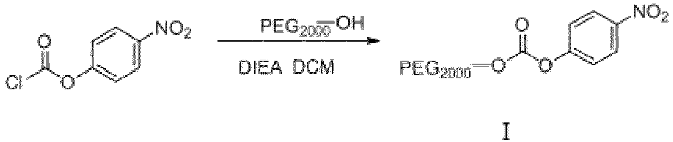

20 g of mPEG2000 is dissolved in 100 mL of DCM, with addition 8 ml of diisopropylethylamine (DIEA) thereto dropwise in an ice bath. The system is colorless and transparent. 8 g of 4-Nitrophenyl chloroformate is dissolved in 50 mL of DCM, and added into the above solution dropwise under the ice bath. After dropping, the system slowly rises to a room temperature. At this time, there is no obvious change in the system, and the reaction is carried out overnight.

Separation and purification: the system is bright yellow and slightly turbid, filtered, and then spun to dry to obtain a yellow viscous liquid. 200 mL of ethyl acetate (EA) is first added into the system and stirred vigorously at a room temperature, the yellow liquid gradually turns into a white solid which is dispersed in the system, the liquid turns into bright yellow, and is added 100 mL of Et₂O dropwise while stirring, pulped at the room temperature for 0.5 h, and stirred to obtain a white solid. The white solid is transferred to a beaker, 200 mL of EA is first added while stirring, then 100 mL of Et₂O is dropped after 15min, pulping is performed for 0.5 h, suction filtration is performed to obtain a white solid, and the solid is dried under a reduced pressure to obtain 16.1 g of the white solid (polymer I).

Reaction B:

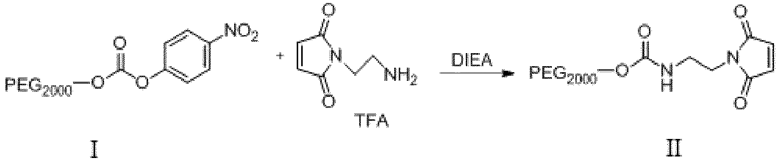

0.65 g of DIEA is weighed and put into a round-bottom flask, a DCM solution is added under an ice bath, then 0.47 g of Maleic-NH₂·TFA(N-(2-aminoethyl)maleimide trifluoroacetate) is added with stirring for 0.5 h, finally 4.3 g of the polymer I is added into the DCM solution dropwise, and it is naturally warmed overnight after the completion of dropping,. The next day, suction filtration is performed, and a filtrate is spun to dry to obtain a yellow oily substance. Pulping is performed twice with 300 mL of a liquid with EA/tert-methyl ether=2/1, and the product is dried under a reduced pressure to obtain 3.9 g of a white solid (polymer II).

### Embodiment 9

### Synthesis of a PEG derivative

Preparation of the polymer I is the same as Embodiment 6.

Reaction C:

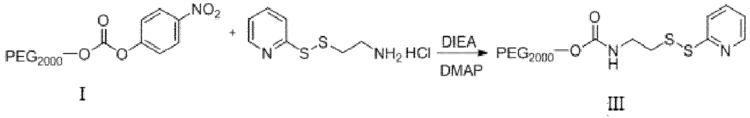

0.65 g of DIEA and 0.12 g of DMAP(4-dimethylaminopyridine) are weighed and put into a round-bottom flask, a DCM solution is added under an ice bath, then 0.45 g of S-(2-aminoethylthio)-2-thiopyridine (Py-SS-NH₂) is added for stirring of 0.5 h, finally the DCM solution with 4.3 g of the polymer I is added dropwise, and it is naturally warmed overnight after the completion of dropping. The next day, suction filtration is performed, and a filtrate is spun to dry to obtain a yellow oily substance. Pulping is performed twice with 300mL of a liquid with EA/tert-methyl ether=2/1, and the product is dried under a reduced pressure to obtain 4.2 g of a white solid (polymer III).

### Embodiment 10

### Synthesis of an intermediate A

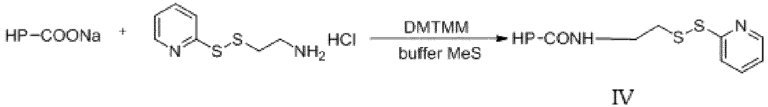

2.88g of enoxaparin sodium (HPCOONa) is dissolved in 10 mL of a MeS buffer solution, and activated for 10min by adding 4.14 g of 4-(4,6-Dimethoxy-1,3,5-triazin-2-yl)-4-methyl morpholinium chloride (DMTMM), then 3.34 g of Py-SS-NH₂·HCl that is dissolved in the 10 mL of MeS buffer solution is added into the system dropwise for reaction of 24 h, thereafter, it is dialyzed for 3 days and lyophilized, to yield 1.5 g of a product (polymer IV).

Preparation of the MeS buffer solution: 2 g of sodium hydroxide is weighed, dissolved in 20 mL of purified water, cooled for later use. 9.8 g of quinoline-8-sulfonic acid is weighed and dissolved in 250 mL of purified water, and the sodium hydroxide solution is added dropwise to adjust the pH to 5.5, and a metered volume is made to 500 mL.

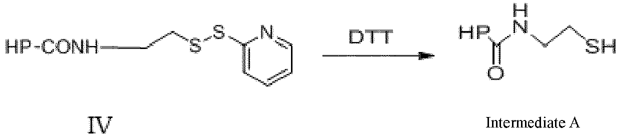

1.5 g of the polymer IV is dissolved in water, 1.5 g of dithiothreitol (DTT) is added at a room temperature, reaction is carried out overnight, dialysis is performed with a semipermeable membrane of 1KDo for three days since the next day, and lyophilization is performed to obtain 1.1 g of a white solid (intermediate A).

### Embodiment 11

### (1) Synthesis of a pemetrexed derivative

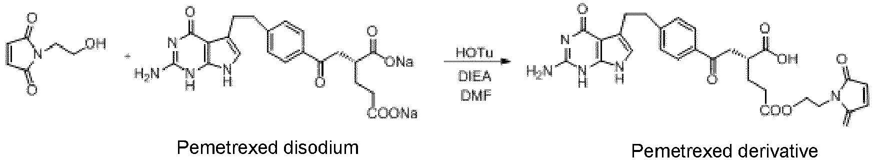

Pemetrexed disodium (2 mmol, 0.95 g), Maleic-NH₂·TFA(N-(2-aminoethyl)maleimide) (2.2 mmol, 0.31 g), and HOTu(O-[(Ethoxycarbonyl)cyanomethylenamino]-N,N,N',N'-tetramethyluronium hexafluorophosphate (2.6 mmol, 0.99 g) are weighed and put into a round-bottom flask, and after substituted nitrogen protection, the flask is wrapped with tin foil paper for light shielding. DMF(15 mL) and DIEA (diisopropylethylamine) (4 mmol, 0.51 g) are added, and stirred at a room temperature overnight. The next day, after addition of 25 mL dichloromethane, it is treated 3 times by washing-liquid separation with a saturated sodium chloride solution. The yellow turbid liquid obtained is spun to dry to a semi-solid state, pulping is performed with tertiary methyl ether/ethyl acetate (25 mL, tertiary methyl ether/ethyl acetate=10/1) in an ice bath for three times, precipitation and drying under a reduced pressure are performed after filtration to obtain a yellow solid (1.03 g).

Pemetrexed is sensitive to light, so raw materials and reaction processes should be kept away from light. There are two carboxyl groups in a pemetrexed molecule. In principle, both of them can participate in the reaction. However, one of the carboxyl groups has a chiral substituent at the α position, and the steric hindrance is somewhat larger, thereby decreasing the activity, so that the other carboxyl group reacts preferentially. It is the same for synthesis of nanomicelles of raltitrexed and methotrexate dihydrate.

### (2) Synthesis of a pemetrexed-PEGylated heparin nanomicelle

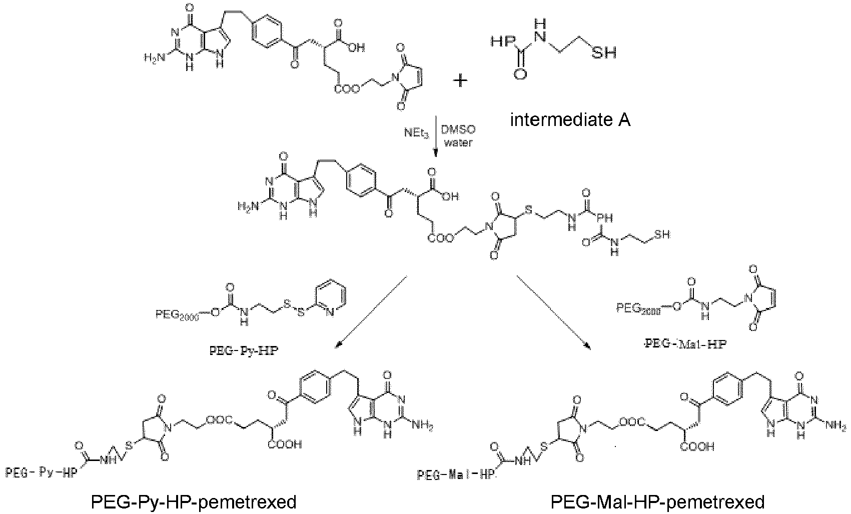

0.2 g of the intermediate A is dissolved and clarified with 4 mL of water, and then 12 ml of DMSO is added. The system remains clear and emits a lot of heat, and is wrapped with a tin foil. 40 mg of the pemetrexed derivative is dissolved in 4 mL of DMSO to obtain a yellow solution which is added dropwise into the system, and a catalytic amount of NEt₃ is added, and stirred overnight.

The solution from the previous step is divided into two duplicates, 0.4 g of corresponding Py-PEG (polymer III) and 0.4 g of Mal-PEG (polymer II) are added respectively, and the reaction is performed overnight at a room temperature by keeping away from the light. Dialysis with a semipermeable membrane of 3.5 KDo is performed on the system for three days, and the product is lyophilized to obtain a yellow solid (with PEG-Py-HP-pemetrexed of 0.25 g; PEG-Mal-HP-pemetrexed of 0.29 g).

PEG-Py-HP-pemetrexed HNMR (D₂O+D-DMSO): 1.9 (the middlemost CH₂ between two carboxyl groups), 2.6-2.7 (carboxyl ortho CH), 2.8-3.0(SCH₂CH₂N, CH₂CH₂ between two aromatic rings), 3.0-3.3 (heparin sodium sugar ring hydrocarbon), 3.4-3.7 (Methylene hydrogen in PEG), 3.8 (CH₃O-), 6.6-6.9 (benzene ring CH, pyrrole CH).

Using UV(SP-1920UV, Shanghai Spectrum instruments Co., Ltd.), the drug loading capacity is measured as 4.25%.

PEG-Mal-HP-pemetrexed HNMR (D₂O+D-DMSO): 1.9 (the middlemost CH₂ between two carboxyl groups), 2.6-2.7 (carboxyl ortho CH), 2.7-2.8(CO-CH₂, CH₂CH₂ between two aromatic rings), 2.9 (CH₂ connected to NH), 3.0-3.3 (heparin sodium sugar ring hydrocarbon), 3.4-3.7 (Methylene hydrogen in PEG), 3.8 (CH₃O-), 3.9 (S-CH₂-CO), 6.6-6.9 (benzene ring CH, pyrrole CH).

Using UV(SP-1920UV, Shanghai Spectrum instruments Co., Ltd.), the drug loading capacity is measured as 5.49%.

### Embodiment 12

Synthesis of a raltitrexed-PEGylated heparin nanomicelle

The synthetic method is the same as that of Embodiment 11 with raltitrexed as a raw material.

Raltitrexed-PEG-Py-HPHNMR (D₂O+D-DMSO): 1.9 (the middlemost CH₂ between two carboxyl groups), 2.6-2.7 (carboxyl ortho CH), 2.7-2.8(CO-CH₂, CH₂CH₂ between two aromatic rings), 2.9 (CH₂ connected to NH), 3.0-3.3 (heparin sodium sugar ring hydrocarbon), 3.4-3.7 (Methylene hydrogen in PEG), 3.8 (CH₃O-), 3.9 (S-CH₂-CO), 4.4 (CH₂ between aromatic ring and N), 6.2-7.9 (benzene ring CH, thiophene ring CH). Using UV(SP-1920UV, Shanghai Spectrum instruments Co., Ltd.), the drug loading capacity is measured as 4.51%.

Raltitrexed-PEG-Mal-HP HNMR (D₂O+D-DMSO): 1.9 (the middlemost CH₂ between two carboxyl groups), 2.6-2.7 (carboxyl ortho CH), 2.8-3.0 (SCH₂CH₂N, CH₂CH₂ between two aromatic rings), 3.0-3.3 (heparin sodium sugar ring hydrocarbon), 3.4-3.7 (Methylene hydrogen in PEG), 4.4 (CH₂ between aromatic ring and N), 6.2-7.9 (benzene ring CH, thiophene ring CH). Using UV(SP-1920UV, Shanghai Spectrum instruments Co., Ltd.), the drug loading capacity is measured as 5.92%.

### Embodiment 13

Synthesis of a methotrexate -PEGylated heparin nanomicelle
the synthesis method is the same as that in Embodiment 11 with methotrexate dihydrate as a raw material.

Methotrexate -PEG-py-HPHNMR (D₂O+D-DMSO): 1.9 (the middlemost CH₂ between two carboxyl groups), 2.6-2.7 (carboxyl ortho CH), 2.7-2.8(CO-CH₂, CH₂CH₂ between two aromatic rings), 2.9 (CH₂ connected to NH), 3.0-3.3 (heparin sodium sugar ring hydrocarbon), 3.4-3.7 (Methylene hydrogen in PEG), 3.8 (CH₃O-), 3.9 (S-CH₂-CO), 4.4 (CH₂ between aromatic ring and N), 6.9-7.5 (benzene ring CH), 8.7 (heterocyclic ring CH). Using UV(SP-1920UV, Shanghai Spectrum instruments Co., Ltd.), the drug loading capacity is measured as 5.25%.

Methotrexate -Mal-PEG-HPHNMR (D₂O+D-DMSO): 1.9 (the middlemost CH₂ between two carboxyl groups), 2.6-2.7 (carboxyl ortho CH), 2.8-3.0 (SCH₂CH₂N, CH₂CH₂ between two aromatic rings), 3.0-3.3 (heparin sodium sugar ring hydrocarbon), 3.4-3.7 (Methylene hydrogen in PEG), 4.4 (CH₂ between aromatic ring and N), 6.9-7.5 (benzene ring CH), 8.7 (heterocyclic ring CH). Using UV(SP-1920UV, Shanghai Spectrum instruments Co., Ltd.), and the drug loading capacity is measured as 5.76%.

### Embodiment 14

### (1) Synthesis of a bendamustine derivative

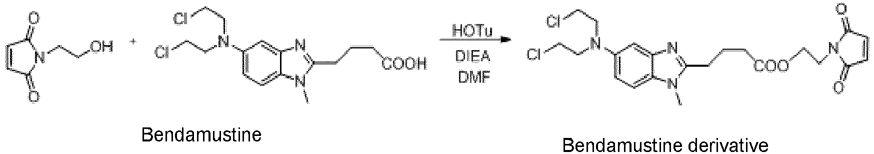

Bendamustine (2 mmol, 0.80 g), Maleic-NH₂·TFA (N-(2-aminoethyl)maleimide) (2.2 mmol, 0.31 g), and HOTu(O-[(Ethoxycarbonyl)cyanomethylen-amine]-N,N,N',N'-tetramethyluronium hexafluorophosphate (2.6 mmol, 0.99 g) are weighed and put into a round-bottom flask. Dichloromethane (25mL) and DIEA (diisopropylethylamine) (4 mmol, 0.51 g) are added, and stirred at a room temperature overnight. The next day, the mixture is treated by washing-liquid separation with a saturated sodium chloride solution for 3 times. An oil phase thereof is dried by anhydrous sodium sulfate, and a yellow liquid is obtained. The liquid is spun to dry to a viscous liquid state, pulped with tertiary methyl ether/ethyl acetate (25 mL, tertiary methyl ether/ethyl acetate=10/1) in an ice bath for three times, and precipitated and dried under a reduced pressure after filtration to obtain a yellow solid (1.08 g).

### (2) Synthesis of a bendamustine-PEGylated heparin nanomicelle

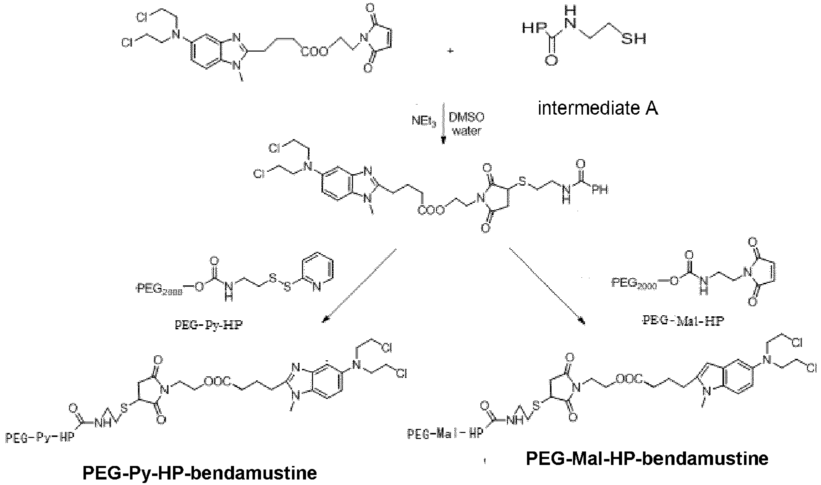

0.2 g of the intermediate A is dissolved and clarified with 4mL of water, and then 12 ml of DMSO is added. The system remains clear and emits a lot of heat, and is wrapped with a tin foil. 40 mg of the bendamustine derivative is dissolved in 4 mL of DMSO to obtain a yellow solution which is added dropwise into the system, a catalytic amount of NEt₃ is added, and stirred overnight. The solution from the previous step is divided into two duplicates, 0.4 g of corresponding Py-PEG (polymer III) and 0.4 g of Mal-PEG (polymer II) are added respectively, and the reaction is performed overnight at a room temperature by keeping away from the light. Dialysis of the system with a semipermeable membrane of 3.5 KDo is performed for three days, and the product is lyophilized to obtain a light yellow solid (with PEG-Py-HP-bendamustine of 0.25 g; PEG-Mal-HP-bendamustine of 0.29 g).

PEG-Py-HP-bendamustine HNMR (D₂O+D-DMSO): 1.7 (the middlemost CH₂ of benzimidazole and ester group), 2.4 (the ortho CH₂ of carbonyl group in ester group), 2.8-3.0 (SCH₂CH₂N), 3.0-3.3 (heparin sodium sugar ring hydrocarbon), 3.4-3.7 (methylene hydrogen in PEG, CH₂CH₂ between CI and N), 3.8 (CH₃O-), 6.6-7.2 (benzene ring CH).

Using UV(SP-1920UV, Shanghai Spectrum instruments Co., Ltd.), the drug loading capacity is measured as 8.97%.

PEG-Mal-HP-bendamustine HNMR (D₂O+D-DMSO): 1.7 (the middle CH₂ of benzimidazole and ester group), 2.4 (the ortho CH₂ of carbonyl group in ester group), 2.7(CO-CH₂), 2.9 (CH₂ connected to NH) 3.0-3.3 (heparin sodium sugar ring hydrocarbon), 3.4-3.7 (methylene hydrogen in PEG, CH₂CH₂ between CI and N), 3.8 (CH₃O-), 3.9 (S-CH₂-CO), 6.6-7.2 (benzene ring CH).

Using UV(SP-1920UV, Shanghai Spectrum instruments Co., Ltd.), the drug loading capacity is measured as 9.81%.

Raw material sources:
4-dimethylaminopyridine (DMAP) Chengdu Kelong Chemical Reagent Factory
4-(4,6-Dimethoxy-1,3,5-triazin-2-yl)-4-methyl morpholinium chloride (DMTMM) J&K Scientific Co., Ltd.
4-Nitrophenyl chloroformate Tianjin HEOWNS Biochemical Technology Co., Ltd.
DL-dithiothreitol (DTT) Aladdin Biochemical Technology Co., Ltd.
Quinoline-8-sulfonic acid (MeS) Aladdin Biochemical Technology Co., Ltd.
S-(2-aminoethylthio)-2-thiopyridine Shanghai BioChemPartner Co., Ltd.
N-(2-aminoethyl)maleimide trifluoroacetate Aladdin Biochemical Technology Co., Ltd.
Enoxaparin Nanjing King-friend Biochemical Pharmaceutical Co., Ltd.
MeO-PEG2000-OH Aladdin biochemical technology co., Itd

Bulk drugs of pemetrexed disodium, bendamustine, raltitrexed and methotrexate dihydrate are purchased from Aladdin Biochemical Technology Co., Ltd.

### Morphological analysis

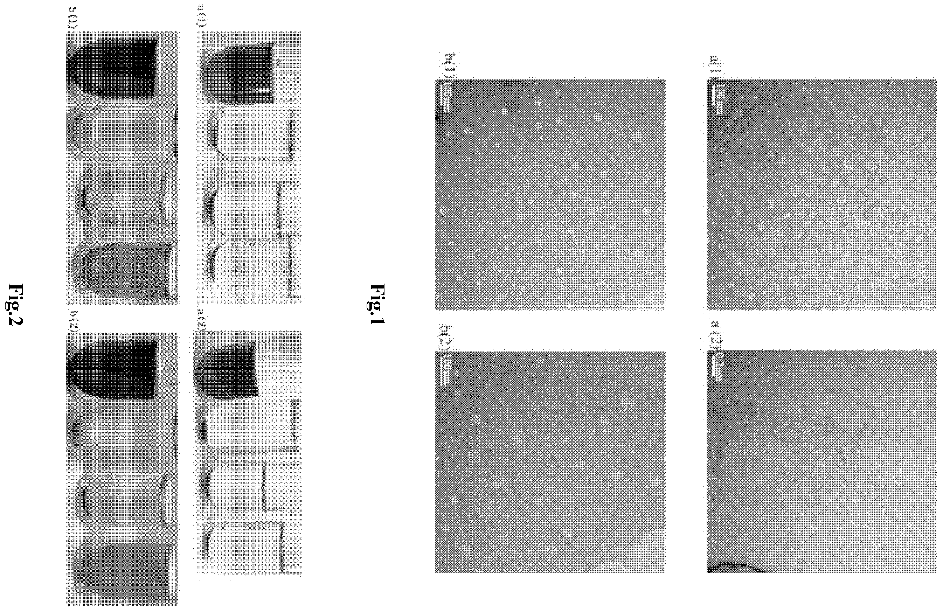

Upon the observation of the appearance of the samples by the field emission transmission electron microscope (TEM) (American FEI Company, Tecnai G2 F20 S-TWIN, Analysis and Testing Center, Sichuan University), the morphological analysis of heparin nano-drug loading systems of pemetrexed and bendamustine is shown in Fig. 1. As investigating the analysis data of electron microscope, the sample is nearly a spherical nanoparticle with a size of about 80-100nm.

### In vitro hemolysis test

Blood biocompatibility is one of the important items to evaluate the biological safety of intravenous injection of MRI contrast agents. 2 mL of fresh blood from healthy BALB/c mice is collected in a heparin tube and centrifuged at a rotating speed of 3000 g for 5min, and isolated at 4°C, to obtain red blood cells (erythrocyte). The resultant red blood cells are suspended in 20% PBS. Four heparin nano-drug loading systems of pemetrexed and bendamustine are added to the above red blood cell solution (50µL) respectively, with the polymer concentrations being set as 0.5 and 4 mg/mL. Incubation is performed at 37°C for 24 h. Then, the above-mentioned red blood cell suspension is centrifuged for 3 min at a rotating speed of 3000 g, so as to get the supernatant, which is detected for the absorbance of 540 nm by a multifunctional microplate reader (BioTek, EON). In the experiment, it takes PBS as negative control and purified water as positive control.

The results are shown in Fig. 2 (from left to right: blood sample, PBS, 0.5 mg/mL, 4 mg/mL). At 37°C, the red blood cells of normal mice are incubated with each nano-drug loading system for 24 hours. After centrifugation, it is demonstrated that all the nanomicelles do not cause hemolysis and have good biocompatibility, due to no distinct red of the supernatant.

### Evaluation of anti-tumor effects

### In vivo experiment of breast cancer cells

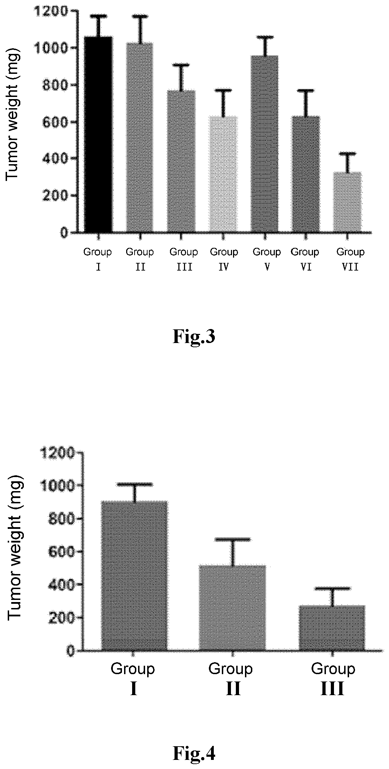

Anti-tumor effects of nanoparticles in vivo are studied in BALB/c mice with 4T1 breast cancer xenograft tumors. Groups represent normal saline (Group I), free pemetrexed disodium (Group II), PEG-Py-HP-pemetrexed (Group III), PEG-Mal-HP-pemetrexed (Group IV), free bendamustine (Group V), PEG-Py-HP-bendamustine (Group VI) and PEG-Mal-HP-bendamustine (Group VII), which are administrated via caudal veins at a dosage of 4 mg/kg, once every two days, with a total of 5 times. As shown in Fig. 3, the tumor development is detected on the second day after administration. Compared with normal saline group, it discovers that all groups have the anti-tumor activity. Based on grouped investigation, the tumor cells in the nanoparticle treatment group all contracted obviously, and the therapeutic effect is better than that of the corresponding free drug groups. These results show that nanoparticles have a better anti-tumor effect than free drugs in vivo.

### In vivo experiment of non-small cell lung cancer

A549 cells in a logarithmic growth stage are inoculated subcutaneously in the necks and backs of nude mice. Groups represent free pemetrexed disodium (Group I), PEG-Py-HP-pemetrexed (Group II) and PEG-Mal-HP-pemetrexed (Group III), which are administrated via caudal veins at a dosage of 4 mg/kg, once every two days, with a total of 5 times. As shown in Fig. 4, the tumor development is detected on the second day after administration. The results show that nanoparticles have a better anti-tumor effect than free drugs in vivo.

### In vivo experiment of multiple myeloma

Thirty 6-week-old mice are divided into three groups, which are administrated with free bendamustine (Group I), PEG-Py-HP-bendamustine (Group II) and PEG-Mal-HP-bendamustine (Group III) respectively. Male and female animals in each group are halved, and there is no statistically significant difference in body weight among animals in each group. The dosage of free bendamustine group is 0.1 ml/kg body weight/day, and that of PEG-Py-HP-bendamustine group and PEG-Mal-HP-bendamustine group is 20 mg/kg (body weight/day).

One week after injection of 1,000,000 5T33 multiple myeloma cells into the tail veins of mice, drugs are administered subcutaneously according to the above scheme, for three times a week. During the administration period, the animals are weighed every day, and the dosage on that day is determined according to the weight, and the animals are continuously administered until the death of the mice. The venous blood of mice is collected and stored every week, and the time of death of mice is recorded.

Experimental results show that subcutaneous injection of PEG-Py-HP-bendamustine and PEG-Mal-HP-bendamustine could significantly prolong the survival time of mice and reduce the tumor load in mice serum (mouse LGg2b concentration). According to the statistical test, there is significant difference in the survival time of mice when free bendamustine group is compared with heparin nano-drug loading system (P<0.05), as shown in Table 1. There is significant difference in the tumor load in serum of mice when the free bendamustine group is compared with heparin nano-drug loading system (P<0.05), as shown in Table 2.

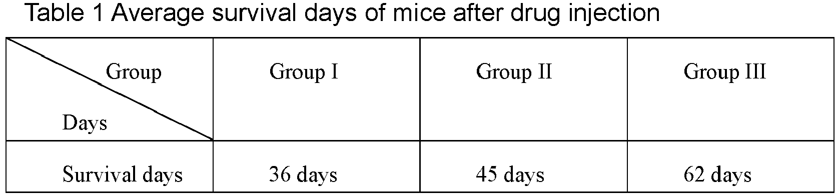

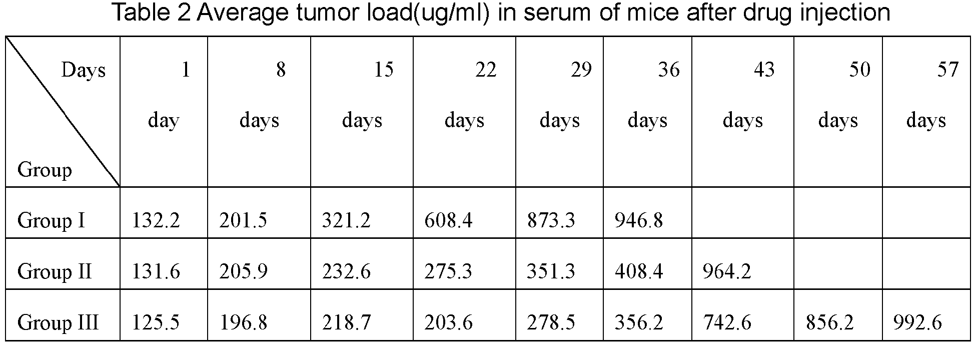

### In vivo toxicity evaluation

Healthy female BALB/c mice aged 6 to 8 weeks (weighing about 20±2 g) are randomly divided into 4 groups with 7 mice in each group, and each mouse is labeled. Then, drugs (200 pL) are injected into mice via tail vein injection, which are four kinds of heparin nanoparticles composed of pemetrexed and bendamustine synthesized by the present invention and normal saline as the control group respectively, wherein the injection dosage is uniformly 20 mg/kg, and the injection is carried out once a day, for 10 times in total. Weights of mice are recorded every two days and behaviors of the mice are observed, with the first-day weight set as 100%. 19 days later, the mice are euthanized and blood of the mice is collected for analysis.

During the overall study of treatment, there are generally good tolerance for repetitive injections of nanoparticles, and the mice did not show any significant weight loss. After the treatment cycle, the mice were euthanized and their blood was collected for routine analysis. As observed, the hematological toxicity (dose-limited toxicity) of nanoparticles was extremely low or even zero. These results demonstrate that carboxylic acid anti-tumor drug nanoparticles administered by intravenous injection are a drug delivery system of low blood toxicity.

The above-mentioned embodiments only illustrate the specific implementation of the present invention, and are descried more specifically and in detail, but the embodiments shall not be construed to limit the scope of the present invention. It should be note that for those ordinary skilled in the art, without departing from the concept of the present invention, several modifications and improvements can be made, which fall within the scope of the present invention.

## Claims

1. A PEGylated heparin nanomicelle loaded with a carboxylic acid anti-tumor drug, **characterized in that** a drug loading system is a conjugate formed by loading a carboxylic acid anti-tumor drug onto a PEGylated heparin molecule; and a specific structure is as follows: 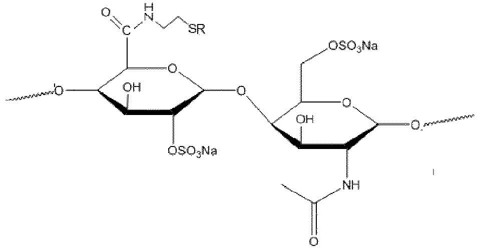 where R is a PEG group and a D group; the PEG group is: 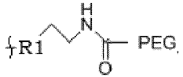 , and this group is an acyl group connected with a hydroxyl group at the end of polyethylene glycol via an ester bond, where R1 is 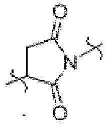 or -S-; the structure of the D group is: 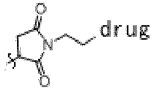 , and this group is connected with a carboxyl group in a drug molecule via an ester bond.

2. The PEGylated heparin nanomicelle loaded with the carboxylic acid anti-tumor drug according to claim 1, **characterized in that** polyethylene glycol (PEG) is mPEG2000.

3. The PEGylated heparin nanomicelle loaded with the carboxylic acid anti-tumor drug according to claim 1, **characterized in that** the D group is connected with an aliphatic carboxyl group in the drug molecule via an ester bond.

4. The PEGylated heparin nanomicelle loaded with the carboxylic acid anti-tumor drug according to claim 1, **characterized in that** the carboxylic acid anti-tumor drug comprises pemetrexed, pemetrexed disodium, raltitrexed or bendamustine.

5. A preparation method of the PEGylated heparin nanomicelle loaded with the carboxylic acid anti-tumor drug of claim 1, **characterized by** comprising the following synthetic scheme:
(1) preparing a PEG derivative; 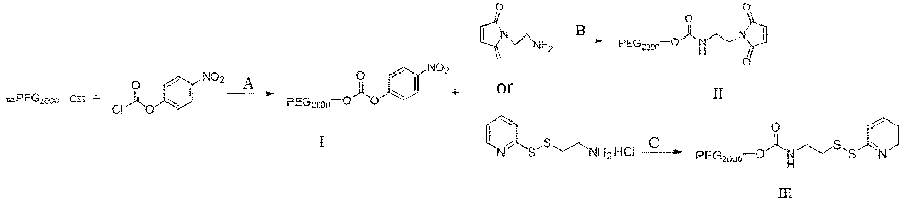
(2) preparing an intermediate A; 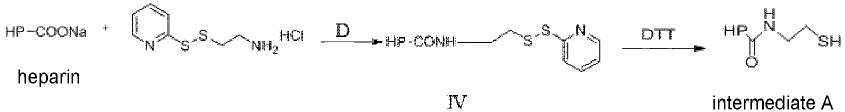
(3) reacting a carboxylic acid drug with Maleic-NH₂ to obtain a derivative of the carboxylic acid drug;
(4) reacting the intermediate A with the prepared derivative of the carboxylic acid drug to obtain a heparin loaded with the carboxylic acid drug;
(5) reacting the heparin loaded with the carboxylic acid drug with the PEG derivative to obtain a PEGylated heparin nanomicelle loaded with the drug.

6. The preparation method of the PEGylated heparin nanomicelle loaded with the carboxylic acid anti-tumor drug according to claim 5, **characterized in that** in the reactions A, B and C of step (1), a catalyst DIEA is added.

7. The preparation method of the PEGylated heparin nanomicelle loaded with the carboxylic acid anti-tumor drug according to claim 5, **characterized in that** in the reaction D, enoxaparin sodium is dissolved in a MeS buffer solution and activated by addition of DMTMM, then S-(2-aminoethylthio)-2-thiopyridine that is dissolved in the MeS buffer solution is added dropwise to the system for reaction, to obtain the intermediate A.

8. The preparation method of the PEGylated heparin nanomicelle loaded with the carboxylic acid anti-tumor drug according to claim 7, **characterized in that** a preparation method of the MeS buffer solution comprises the following steps: weighing quinoline-8-sulfonic acid and dissolving the same in purified water, adding a sodium hydroxide solution dropwise to adjust the pH to 5.5, and making a metered volume to obtain the MeS buffer solution.

9. The preparation method of the PEGylated heparin nanomicelle loaded with the carboxylic acid anti-tumor drug according to claim 5, **characterized in that** in the reaction of step (3), HOTu is added to participate in the reaction.

10. The preparation method of the PEGylated heparin nanomicelle loaded with the carboxylic acid anti-tumor drug according to claim 5, **characterized in that** in the reaction of step (4), the intermediate A reacts with the derivative of the carboxylic acid drug, by addition of triethylamine as a catalyst.

11. The PEGylated heparin nanomicelle loaded with the carboxylic acid anti-tumor drug according to claim 1, **characterized in that** the drug loading capacity of the nanomicelle is 4 wt%-15 wt%.
